Europäisches Patentamt

European Patent Office

Office européen des brevets

(19)

(11) **EP 0 630 650 B1**

(12) **EUROPÄISCHE PATENTSCHRIFT**

(45) Veröffentlichungstag und Bekanntmachung des Hinweises auf die Patenterteilung:
**22.09.1999 Patentblatt 1999/38**

(51) Int. Cl.$^6$: **A61K 33/00**
// (A61K33/00, 31:19)

(21) Anmeldenummer: **94810364.3**

(22) Anmeldetag: **21.06.1994**

(54) **Salpetersäure enthaltendes Haut- und Schleimhautpräparat**

Preparation for skin and mucoses containing nitric acid

Préparation pour la peau et les mucoses contenant de l'acide nitrique

(84) Benannte Vertragsstaaten:
**AT BE CH DE ES FR GB IT LI NL SE**

(30) Priorität: **23.06.1993 CH 188793**

(43) Veröffentlichungstag der Anmeldung:
**28.12.1994 Patentblatt 1994/52**

(73) Patentinhaber: **Solco Basel AG**
**CH-4052 Basel (CH)**

(72) Erfinder:
• **Grabo, Michael**
**CH-4051 Basel (CH)**
• **Stähli, Christian**
**CH-4052 Basel (CH)**
• **Meyer, Rudolf**
**CH-4246 Wahlen (CH)**

• **Glauser, René**
**CH-4053 Basel (CH)**
• **Weiner, Murray**
**Cincinnati, OH 45242 (US)**

(74) Vertreter:
**Zimmermann, Hans, Dr. et al**
**A. Braun Braun Héritier Eschmann AG**
**Holbeinstrasse 36-38**
**4051 Basel (CH)**

(56) Entgegenhaltungen:
**EP-A- 0 026 532**

• **N.F. MULLER, (ED.), 'EUROPEAN DRUG INDEX',**
**2ND EDITION, 1992, ELSEVIER, AMSTERDAM.**

**Beschreibung**

Die Erfindung betrifft ein neues Präparat auf der Basis einer wässrigen, Nitrat-Reduktionsprodukte enthaltenden Salpetersäurelösung zur lokalen Behandlung von Haut- und Schleimhautveränderungen, insbesondere von oberflächlichen, gross- und kleinflächigen Haut-und Schleimhautveränderungen benigner Art und von oberflächlichen Infektionen der Haut und Schleimhaut, sowie ein Verfahren zur Herstellung dieses Präparates.

Schon seit Jahrhunderten werden zum "Ausbrennen" von Hautfehlern, Warzen und dergleichen ätzende Präparate, insbesondere starke Säuren, verwendet. Dabei haben sich vor allem Salicylsäure, konzentrierte Formen der Salpetersäure und gewisse Halogenessigsäuren einen mehr oder weniger spezifischen Platz im Arzneimittelschatz der Dermatologen verschafft, während Salzsäure selten verwendet wird. Behandlungen mit ätzenden Präparaten sind im allgemeinen schmerzhaft und hinterlassen bekanntlich öfters mehr oder weniger hässliche Narben.

Zur Beseitigung von Warzen wurden ferner auch Milchsäure, Oxalsäure und Essigsäure vorgeschlagen. Die keratolytische Wirkung dieser Säuren ist jedoch gering, und sie werden daher fast immer in Kombination mit Salicylsäure verwendet (Deutsche Auslegeschrift 1266448).

Andererseits sind im 19. Jahrhundert zur Beseitigung von Warzen und anderen Hautfehlern auch verschiedene Metallsalze vorgeschlagen worden, darunter Kupfersalze wie das Acetat oder das Sulfat, Bleisalze in Kombination mit Zinksulfat, Kupfersulfat zusammen mit Essig und ferner Antimon-, Arsen-, Chrom-, Quecksilber-, Silber-, Zink- und Cadmiumsalze. Von diesen Salzen hat lediglich Zinkchlorid in Kombination mit Trichloressigsäure - Methode von Mohs - zur Behandlung von Hautkrebs eine vorübergehende Bedeutung erlangt.

In EP-A-26532 wurde zur lokalen Behandlung von oberflächlichen Haut- und Schleimhautveränderungen die Verwendung einer 6 - 10 M wässrigen Salpetersäurelösung empfohlen, welche Metallnitrit oder salpetrige Säure in einer Menge enthält, die 0,01 bis 5 mg, vorzugsweise 0,1 bis 0,5 mg Nitrit pro ml Lösung entspricht. Wie in EP-A-26532 offenbart wird, ergeben solche Präparate - gemessen an der Verfärbung der Haut - eine ähnlich rasche chemische Reaktion wie konzentrierte Salpetersäure, jedoch ohne die kräftige ätzende, alle Gewebe undifferenziert auflösende Wirkung dieser und anderer starker Säuren. Unter der Einwirkung der Präparate werden nämlich die integumentalen Proteine in situ denaturiert, und die anatomische Struktur wird ohne Verletzung intravital fixiert (Mumifizierung).

Es erwies sich für die Zusammensetzung und Wirkung der vorbekannten Präparate als besonders vorteilhaft, die Präparate durch Zugabe oxidierbarer organischer Carbonsäuren zur Salpetersäure herzustellen oder allenfalls den Präparaten solche oxidierbaren organischen Carbonsäuren zuzusetzen. Die oxidierbaren Carbonsäuren, wie Oxalsäure, Milchsäure, Glykolsäure, Glyoxylsäure, Aepfelsäure und dergleichen, bilden mit der Salpetersäure eine Kaskade von Umsetzungsprodukten, insbesondere Nitrat-Reduktionsprodukte wie nitrose Gase und salpetrige Säure und ferner Kondensationsprodukte wie O-Nitryl- und O-Nitrosylderivate. Die nach dieser oxidativen Methode erhaltenen Präparate ergeben insbesondere eine verbesserte Wirksamkeit über längere Zeitspannen. Es ist nicht im einzelnen bekannt, welche der Umsetzungsprodukte für die klinisch nützlichen Eigenschaften primär verantwortlich sind, wobei jedoch die Wirksamkeit verloren geht, wenn der Gehalt der als Nitrit messbaren Umsetzungsprodukte zu stark abfällt.

Die Umsetzung der oxidierbaren organischen Carbonsäuren mit 6 - 10 M Salpetersäure erfolgt unter Bildung von nitrosen Gasen, Kohlendioxid etc. Gemäss der Offenbarung von EP-A-26 532 ist es daher wichtig, dass Präparate, die nach dieser Methode hergestellt werden, nicht hermetisch verschlossen werden, sondern in einem mit lockerem Verschluss versehenen Gefäss aufbewahrt werden. EP-A-26 532 empfiehlt zu diesem Zweck die Verwendung eines Gemisches von unterschiedlich rasch oxidierbaren Carbonsäuren, beispielsweise die Verwendung eines Gemisches von Brenztraubensäure, Milchsäure und Oxalsäure, um eine Abnahme im NitritGehalt, die infolge Lagerung oder Oeffnen des Gefässes eintreten kann, ausgleichen zu können. Die Praxis zeigt allerdings, dass bei Raumtemperatur die zur Nitrit-Nachbildung benötigte Umsetzung der oxidierbaren Carbonsäuren zu langsam erfolgt.

Entsprechende Präparate werden von der Anmelderin unter den Namen Solcoderm und Solcogyn vertrieben und haben sich in der Praxis gut bewährt, so lange sie eine ausreichende Nitrit-Konzentration aufweisen.

Die vorbekannten Präparate haben jedoch den Nachteil, dass die Reaktionsgeschwindigkeiten der oxidierbaren Carbonsäuren stark temperaturabhängig sind. Infolgedessen kann die Nitrit-Konzentration je nach Lagertemperatur und Lagerzeit erheblichen Schwankungen unterliegen. Eine ausreichende Reproduzierbarkeit bezüglich Zusammensetzung und Wirkung der Präparate ist daher nur gewährleistet, wenn die empfohlene Aufbewahrungstemperatur und die angegebenen Haltbarkeitsdaten möglichst genau beachtet werden. Andernfalls kann die Nitrit-Konzentration unter Umständen so weit abnehmen, dass die Präparate unwirksam werden. Es wurde beobachtet, dass solche unwirksam gewordenen Präparate eine erhöhte Gefahr von Nebenwirkungen aufweisen und beispielsweise zu Ulzerationen auf der gesunden Haut führen können.

Es wurde nun gefunden, dass eine gleich gute und reproduzierbare Wirksamkeit, ohne die Nachteile der vorbekannten Präparate, mit einem neuen Präparat erzielt werden kann, welches überraschenderweise zugleich eine weitere Verringerung der Salpetersäurekonzentration gestattet. Das neue Präparat ermöglicht zudem eine besonders einfache Herstellung aus 1 - 5,5 M wässriger Salpetersäure und einem primären $C_1$-$C_5$-Alkanol in einer Menge von 45 bis 170 mmol pro l Salpetersäure und eine Vereinfachung der Zusammensetzung. Letzteres ist auch deshalb von Vorteil, weil Präparate, die aus vielen Wirksubstanzen bestehen, in der modernen Pharmakologie generell als problematisch erachtet werden.

Das erfindungsgemässe Präparat zur lokalen Behandlung von oberflächlichen Haut- und Schleimhautveränderungen besteht aus 1 - 5,5 M wässriger Salpetersäure, Nitrat-Reduktionsprodukten in einer Konzentration, die 0,1 bis 6 mg Nitrit pro ml Lösung entspricht, und $C_1$-$C_5$-Alkansäure in einer Konzentration von höchstens 170 mmol pro l Lösung. Die erfindungsgemässen Präparate enthalten im allgemeinen mindestens etwa 5 mmol $C_1$-$C_5$-Alkansäure, beispielsweise 45 bis 170 mmol $C_1$-$C_5$-Alkansäure, pro l Lösung. In der Regel sind Präparate bevorzugt, die mindestens etwa 9 und höchstens etwa 90 mmol $C_1$-$C_5$-Alkansäure pro l Lösung enthalten.

Im Rahmen der vorliegenden Erfindungen beziehen sich Angaben zur Nitrit-Konzentration jeweils auf Werte, die nach der von N.G. Bunton, N.T. Crosby und S.J. Patterson in Analyst 94, 585 (1969) beschriebenen Methode durch Umsetzung mit Sulfanilsäure und Naphthyl-1-amin und photometrische Bestimmung erhalten wurden. Bei dieser Methode werden andere, in der Lösung vorhandene Nitrat-Reduktionsprodukte, wie nitrose Gase, in Nitrit übergeführt und ebenfalls erfasst. Die angegebenen Nitrit-Konzentrationen bedeuten somit jeweils die zur Gesamtkonzentration an Nitrat-Reduktionsprodukten äquivalente Nitrit-Konzentration.

Das neue Präparat kann erfindungsgemäss in einfacher Weise dadurch hergestellt werden, dass man 1 - 5,5 M wässrige Salpetersäure mit einem primären $C_1$-$C_5$-Alkanol in einer Menge von 45 bis 170 mmol pro l Salpetersäure reagieren lässt. Ueberraschenderweise wurde nämlich gefunden, dass primäre $C_1$-$C_5$-Alkanole in der wässrigen Salpetersäure schon bei Raumtemperatur oder erhöhter Temperatur rasch und vollständig umgesetzt und dabei in $C_1$-$C_5$-Alkansäuren und Kohlendioxid übergeführt werden unter gleichzeitiger Bildung einer wirksamen Kaskade von Nitrat-Reduktionsprodukten. Die Umsetzung kann vorzugsweise bei einer Temperatur von etwa 20 bis 60°C erfolgen.

Vorzugsweise wird ein $C_2$-$C_5$-Alkanol, wie Aethanol und 1-Propanol, verwendet. Besonders bevorzugt ist Aethanol, welches bereits durch blosses Mischen mit der Salpetersäure bei Raumtemperatur vollständig umgesetzt und dabei in die keratolytisch wirkende Essigsäure (sowie teilweise in Kohlendioxid) übergeführt wird. Die im erfindungsgemässen Präparat vorhandene Alkansäure ist somit vorzugsweise $C_2$-$C_5$-Alkansäure, insbesondere Essigsäure.

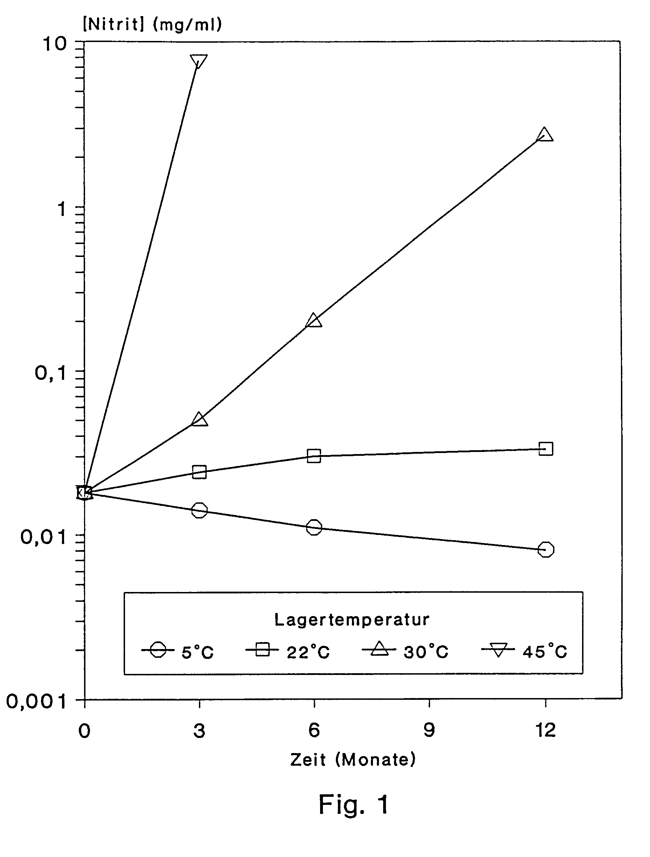

In Fig. 1 bis 5 sind Ergebnisse, die in verschiedenen Versuchsreihen für erfindungsgemässe Präparate und Vergleichspräparate erhalten wurden, graphisch dargestellt.

Fig. 1 zeigt in graphischer Darstellung die Nitrit-Konzentration des vorbekannten Präparates Solcoderm in Abhängigkeit von der Aufbewahrungstemperatur und der Aufbewahrungszeit. Zur Bestimmung dieser Abhängigkeit wurde das Handelspräparat, welches im Ausgangsgemisch 625,2 mg 65%-ige Salpetersäure, 41,5 mg 98%-ige Essigsäure, 57,4 mg Oxalsäure-Dihydrat, 4,5 mg Milchsäure und 48 µg Kupfer-(II)-nitrat-Trihydrat pro ml Lösung enthielt, in hermetisch geschlossenen Glasampullen bei unterschiedlichen Temperaturen über mehrere Monate gelagert und in regelmässigen Abständen die Nitrit-Konzentration gemessen. Wie die in Fig. 1 dargestellten Ergebnisse zeigen, kann die Nitrit-Konzentration je nach Temperatur erheblichen Schwankungen unterliegen, wodurch eine sichere Handhabung deutlich erschwert wird. Insbesondere wird bei niedriger Lagertemperatur von 5°C ein Absinken des Nitrit-Gehaltes und somit ein Verlust der Wirksamkeit beobachtet. Anderseits wird bei Temperaturen von 30°C bzw. 45°C eine starke Zunahme der Nitrit-Konzentration festgestellt, was sich infolge der beschleunigten Umsetzung der Carbonsäuren auf die Haltbarkeit auswirken kann.

Im Gegensatz zum vorbekannten Präparat besitzen die erfindungsgemässen Präparate infolge der raschen und vollständigen Umsetzung eine definierte und reproduzierbare Zusammensetzung, womit Unsicherheiten bezüglich der Wirksamkeit weitestgehend ausgeschlossen werden können.

Offenes Stehenlassen der Präparate führt jedoch infolge Abgabe nitroser Gase sowohl bei den erfindungsgemässen Präparaten als auch bei den vorbekannten Präparaten innert weniger Stunden zu einer deutlichen Abnahme des Gehaltes an Nitrat-Reduktionsprodukten. Die erfindungsgemässen Präparate werden daher vorzugsweise in gasdicht verschlossenen Gefässen, beispielsweise in Glasampullen, aufbewahrt oder allenfalls erst kurz vor der Anwendung hergestellt.

Erfindungsgemässe Präparate, die in gasdicht verschlossenem Gefäss, vorzugsweise in verschlossener Ampulle vorliegen, besitzen eine konstante und im wesentlichen temperaturunabhängige Zusammensetzung und Wirkung, und sie sind praktisch unbeschränkt haltbar. Die Aufbewahrung unter gasdichtem Verschluss wird zudem dadurch wesentlich erleichtert, dass im Unterschied zu den vorbekannten Präparaten keine kontinuierliche Bildung von Kohlendioxid und nitrosen Gasen über einen längeren Zeitraum stattfindet. Die Gefahr, dass bei der Lagerung zu hohe Drücke entstehen können, die bei erhöhten Lagertemperaturen im Extremfall zum Bersten der Ampullen oder beim Oeffnen zu explosionsartigem Austreten der Lösung führen könnten, ist daher bei den erfindungsgemässen Präparaten praktisch ausgeschlossen.

Die Herstellung gasdicht verschlossener Präparate kann grundsätzlich dadurch erfolgen, dass man zuerst, wie oben beschrieben, das Präparat herstellt und dieses dann in einem geeigneten Gefäss gasdicht verschliesst. Vorzugsweise wird jedoch so vorgegangen, dass man die 1 - 5,5 M Salpetersäure und das primäre $C_1$-$C_5$-Alkanol bei einer Temperatur unterhalb der Umsetzungstemperatur, vorzugsweise mindestens 10°C unterhalb Umsetzungstemperatur (z.B. bei etwa 0°C), mischt, das Gemisch in einem geeigneten Gefäss, vorzugsweise in einer Ampulle, gasdicht verschliesst und dann auf Umsetzungstemperatur oder höhere Temperatur erwärmt. Wird Aethanol verwendet, so erfolgt das Mischen der Komponenten vorzugsweise bei einer Temperatur von höchstens 10°C, beispielsweise bei 0 - 5°C, und die anschliessende Umsetzung bei einer Temperatur von vorzugsweise etwa 20 bis 40°C.

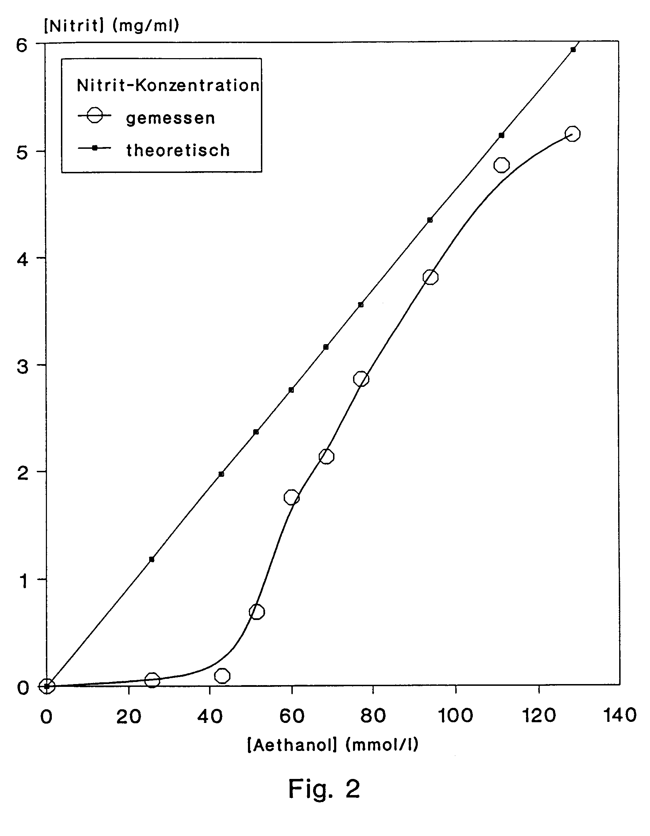

Die Konzentration der durch Umsetzung von primärem $C_1$-$C_5$-Alkanol mit Salpetersäure gebildeten Nitrat-Reduktionsprodukte ist im untersuchten Bereich im wesentlichen unabhängig von der Salpetersäure-Konzentration, wie in den Beispielen 1 und 2 illustriert wird. Sie hängt jedoch in hohem Masse von der verwendeten Alkanol-Konzentration ab, wobei ein nicht-linearer Zusammenhang zwischen der Alkanol-Konzentration und der gemessenen Nitrit-Konzentration gefunden wurde. Wie in Fig. 2 und in den Beispielen 1 und 2 für Aethanol gezeigt wird, werden bei Aethanol-Konzentrationen von weniger als 45 mmol/l nur geringe Mengen an Nitrat-Reduktionsprodukten gebildet, und die gemessenen Nitrit-Konzentrationen liegen im allgemeinen deutlich unterhalb einer Konzentration von 0,1 mg/ml, die den Beginn der therapeutischen Wirksamkeit reflektiert. Bei weiterer Erhöhung der Aethanol-Konzentration erfolgt jedoch ein steiler Anstieg der gemessenen Nitrit-Konzentration. Die Ursache für diesen Effekt ist nicht bekannt. Er eröffnet jedoch die Möglichkeit, durch Umsetzung eines $C_1$-$C_5$-Alkanols mit Salpetersäure wirksame Präparate zu erhalten, wenn das Alkanol in einer Konzentration von mindestens 45 mmol/l eingesetzt wird, und er bietet darüber hinaus die Möglichkeit zur Uebersättigung der Lösung mit nitrosen Gasen, wenn die Alkanol-Konzentration weiter erhöht wird.

Die erfindungsgemässen Präparate erwiesen sich in dem in Beispiel 3 beschriebenen Haar-Test als gleichermassen wirksam wie ein in gleicher Weise mit 6,4 M Salpetersäure hergestelltes Präparat und wie das Handelspräparat Solcoderm. In allen Fällen wurde eine rasche Gelbfärbung bewirkt und die Zersetzung der Haare unterdrückt, während ein ungenügender Alkanol-Zusatz oder reine Salpetersäure zur Zersetzung der Haare führten. Die vorliegende Erfindung ermöglicht daher, entgegen den Erkenntnissen von EP-A-26 532, überraschenderweise eine Verringerung der Salpetersäure-Konzentration auf deutlich weniger als 6 mol/l unter Beibehaltung der Wirksamkeit.

Um allfällige Unterschiede in der Wirksamkeit der Präparate genauer erfassen zu können, wurde der in Beispiel 4 beschriebene Pepsin-Test als Modell für die erste Reaktion der Mumifizierung entwickelt. Fig. 3 zeigt die im Pepsin-Test für Proben 1-6 gemessenen optischen Dichten (OD) in Abhängigkeit von der Zeit. Die "Steigung b" der Kurven wurde als Mass für das Wirkungsmodell (Schnelligkeit der Verfärbung) verwendet. Fig. 4 zeigt die Abhängigkeit der Steigung b im Pepsin-Test von der verwendeten Aethanol-Konzentration im Falle von 5,4 M und 6,4 M Salpetersäure. Fig. 5 zeigt die Abhängigkeit der Steigung b im Pepsin-Test von der Salpetersäure-Konzentration bei Verwendung einer konstanten Aethanol-Konzentration von 60 mmol/l (0,35 Vol.-%).

Die unter Verwendung einer Alkanol-Konzentration von mindestens 45 mmol/l hergestellten Präparate erwiesen sich auch im Pepsin-Test als ausreichend wirksam. Ueberraschenderweise zeigen die Ergebnisse jedoch, dass die Schnelligkeit der Verfärbung im Pepsin-Test sowohl mit steigender Salpetersäure-Konzentration als auch mit steigender Alkanol-Konzentration zunimmt. Aus diesem Befund folgt, dass eine durch Verringerung der Salpetersäure-Konzentration bedingte Verlangsamung der Verfärbung gewünschtenfalls weitgehend durch eine Erhöhung der Alkanol-Konzentration - die zu einer entsprechenden Erhöhung der Konzentration an Nitrat-Reduktionsprodukten führt (vgl. Fig. 2) - kompensiert werden kann.

Das erfindungsgemässe Herstellungsverfahren führt zu einer vollständigen Umsetzung der Alkanole, wobei die entsprechenden Alkansäuren und (insbesondere bei Verwendung von Methanol oder Aethanol) teilweise auch Kohlendioxid gebildet wird.

Die Alkanole werden zweckmässigerweise in einer Konzentration von höchstens etwa 170 mmol/l, vorzugsweise höchstens etwa 130 mmol/l eingesetzt. Hierdurch wird ein zu hoher Druck vermieden, wenn die Präparate unter gasdichtem Verschluss hergestellt werden. Im Hinblick auf die Ergebnisse im Pepsin-Test wird in der Regel bevorzugt eine Alkanol-Konzentration von etwa 60 bis 90 mmol/l verwendet.

Dementsprechend enthalten die erfindungsgemässen Präparate $C_1$-$C_5$-Alkansäure in einer Konzentration von höchstens 170 mmol/l, vorzugsweise etwa 9 bis 90 mmol/l, und die als Nitrit gemessene Konzentration an Nitrat-Reduktionsprodukten beträgt zweckmässigerweise etwa 0,1 bis etwa 6, vorzugsweise etwa 1 bis 5, besonders bevorzugt etwa 2 bis 4 mg Nitrit pro ml.

Um die Bildung von Nitrat-Reduktionsprodukten in Form von nitrosen Gasen zu fördern, wird im Falle der Herstellung bzw. Aufbewahrung in gasdicht verschlossenen Gefässen die Grösse der Gefässe vorzugsweise so gewählt, dass das Verhältnis des Flüssigkeitsvolumens zum Gesamtvolumen des Gefässes höchstens 1:2, besonders bevorzugt etwa 1:10 bis 1:5, beträgt. Beispielsweise können die erfindungsgemässen Präparate in Mengen von je etwa 0,1 bis 0,2 ml, die für eine einzelne Behandlung in der Regel ausreichen, in 1 ml-Glasampullen hergestellt und gelagert werden. Dadurch entfällt die Entsorgung überschüssiger Lösung und die Gefahr, dass unwirksam gewordene Lösungen angewandt werden könnten, praktisch völlig.

Wie der Haut-Test im Beispiel 5 zeigt, führen die erfindungsgemässen Präparate selbst im oberen Bereich der Salpetersäure-Konzentration abgesehen von einer leichten Rötung und sehr geringer Blasenbildung, die innert weniger Stunden wieder verschwindet, zu keinen Nebenwirkungen. Selbst wenn infolge unsachgemässer Handhabung der Gehalt an Nitrat-Reduktionsprodukten völlig abgebaut würde, blieben die Nebenwirkungen dank der niedrigen Salpetersäure-Konzentration vergleichsweise gering. Ein analog hergestelltes Präparat mit einer Salpetersäure-Konzentration von 6,4 mol/l ergab geringfügig höhere Nebenwirkungen und erwies sich aber ebenfalls als besser als das Handelspräparat Solcoderm. Bei beiden Vergleichspräparaten besteht jedoch die Gefahr, dass infolge unsachgemässer Handhabung stärkere Nebenwirkungen, z.B. offene Wunden oder Ulzerationen, auftreten können. Erfindungsgemäss wird daher die Salpetersäure zweckmässigerweise in einer Konzentration von höchstens 5,5 mol/l verwendet.

Die erfindungsgemässen Präparate ermöglichen somit bei etwa gleich guter Wirksamkeit wie die vorbekannten Präparate eine erheblich sicherere Anwendung, da geringere Nebenwirkungen auftreten, eine definierte Zusammensetzung und Wirksamkeit vorliegt und das Nebenwirkungsrisiko selbst bei unsachgemässer Handhabung relativ gering bleibt.

Die erfindungsgemässen Präparate können in der Kosmetik und in der Medizin angewendet werden und eignen sich für alle in EP-A-26 532 angegebenen Anwendungsgebiete und Indikationen. Sie sind insbesondere indiziert für die topische Behandlung oberflächlicher, benigner Hautveränderungen, wie Verrucae (z.B. Verrucae vulgares und Verrucae plantares), benigne Naevi, seborrhoische und aktinische Keratosen und Condylomata und oberflächlicher benigner Schleimhautveränderungen, z.B. benigner Zervix-Läsionen, wie Portioektopien (beispielsweise Ektropium, Erythroplakie und Pseudoerosion), Umwandlungszonen, Ovula Nabothi, Zervikalkanal-Polypen, Condolymata und postoperative Granulome.

Aufgrund ihres sehr geringen Nebenwirkungsrisikos eignen sich die erfindungsgemässen Präparate im Gegensatz zu den in EP-A-26 532 beschriebenen Präparaten nicht nur zur Behandlung von kleinflächigen Haut- und Schleimhautveränderungen, sondern auch zur Behandlung von grossflächigen, flachen Läsionen und Infektionen der Haut und Schleimhaut, insbesondere auch zur Behandlung von Pilzerkrankungen der Haut und Schleimhaut, wie z.B. Fusspilz und Nagelpilz.

Alle erfindungsgemässen Präparate eignen sich grundsätzlich für die genannten Indikationen. Zur Behandlung von grossflächigen, flachen Läsionen und von Pilzerkrankungen der Haut und Schleimhaut werden aber im allgemeinen bevorzugt Präparate verwendet, die eine Salpetersäure-Konzentration von 1 bis 4 mol/l aufweisen. Zur Behandlung von hyperplastischen, d.h. erhöhten, deutlich abgegrenzten Haut- und Schleimhautveränderungen werden hingegen bevorzugt Präparate mit einer Salpetersäure-Konzentration von 4 bis 5,5 mol/l verwendet.

Die erfindungsgemässen Präparate können in gleicher Weise wie in EP-A-26 532 beschrieben durch topisches Auftragen oder andere Formen der Lokalbehandlung auf bzw. in die pathologisch veränderten Gewebeteile appliziert werden. Als Applikator kann vorzugsweise ein spitzes Holzstäbchen oder ein dünnes, poröses Kunststoffstäbchen dienen oder zur Behandlung grösserer Flächen ein Pinsel.

Die Erfindung wird in den nachfolgenden Beispielen weiter veranschaulicht. Raumtemperatur bedeutet jeweils eine Temperatur von 22°C. Nitrit-Konzentrationen wurden jeweils nach der in Analyst 94, 585 (1969) beschriebenen Methode durch Umsetzung mit Sulfanilsäure und Naphthyl-1-amin bestimmt und beziehen sich jeweils auf die Gesamtkonzentration der als Nitrit messbaren Nitrat-Reduktionsprodukte.

Beispiel 1

1 Volumenteil 70-prozentige Salpetersäure (15,7 mol/l) und 1,907 Volumenteile Wasser wurden in separaten Gefässen im Eisbad auf unter 5°C vorgekühlt. Danach wurde das kalte Wasser langsam und unter Rühren und Kühlung mit der kalten Salpetersäure versetzt und die Lösung (5,4 M Salpetersäure) im Eisbad noch 15 Minuten gemischt. Anschliessend wurde die Lösung mit Aethanol in einer Menge von 60 mmol pro l Lösung versetzt und gerührt. Mittels einer Ampulliermaschine wurden jeweils 0,2 ml der Lösung in 1 ml DIN-Ampullen mit Brechring abgefüllt und die Ampullen sofort verschmolzen. Die Lösung wurde während der Aethanol-Zugabe und der ganzen Abfülldauer im Eisbad bei 0-2°C gehalten. An Proben, die während 24 Stunden bei 0-2°C gehalten wurden, wurde keine Reaktion beobachtet, so dass auch die Herstellung grosser Chargen nach dieser Methode gewährleistet ist.

Die verschlossenen Ampullen wurden auf Raumtemperatur erwärmen gelassen, wobei eine spontane und vollständige Umsetzung des Aethanols unter Bildung von Essigsäure und nitrosen Gasen einsetzte. Nach 24 Stunden lag der mittels Hochdruck-Flüssigchromatographie bestimmte Aethanol-Restgehalt jeweils deutlich unter 100 ppm. Das erhaltene, ampullierte Präparat, welches aus wässriger Salpetersäure, Nitrat-Reduktionsprodukten und Essigsäure bestand, wies ein Säureäquivalent von 5,50 mol/l und eine Konzentration an Nitrat-Reduktionsprodukten, gemessen als Nitrit, von 1,41 mg Nitrit pro ml Lösung auf.

In analoger Weise, aber unter Verwendung unterschiedlicher Aethanol- und Salpetersäure-Konzentrationen wurden die in Tabelle 1 aufgeführten Präparate hergestellt und die in der Tabelle angegebenen Nitrit-Konzentrationen gemessen.

Tabelle 1

| Aethanol-Konzentration (mmol/l) | Salpetersäure-Konzentration (mol/l) | Nitrit-Konzentration (mg/ml) |
|---|---|---|
| 43 | 5,4 | 0,08 |
| 43 | 6,4 | 0,09 |
| 60 | 4,9 | 1,61 |
| 60 | 5,4 | 1,41 |
| 60 | 6,4 | 1,75 |
| 86 | 5,4 | 3,20 |
| 129 | 5,4 | 5,33 |
| 129 | 6,4 | 5,14 |

Beispiel 2

In analoger Weise zu Beispiel 1 wurde aus 1 Volumenteil 70-prozentiger Salpetersäure und 1,453 Volumenteilen Wasser eine 6,4 M Salpetersäurelösung hergestellt und diese mit Aethanol in einer Menge von 60 mmol pro l Lösung versetzt, ampulliert und auf Raumtemperatur erwärmen gelassen. Das erhaltene Präparat hatte ein Säureäquivalent von 6,64 mol/l und eine Konzentration an Nitrat-Reduktionsprodukten, gemessen als Nitrit, von 1,75 mg Nitrit pro ml Lösung.

Die Konzentration an Nitrat-Reduktionsprodukten erwies sich im Rahmen der Messgenauigkeit als weitgehend unabhängig von der Salpetersäure-Konzentration (vgl. Beispiel 1 und Tabelle 1), wie aufgrund des hohen Salpetersäureüberschusses und der vollständigen Oxidation des Aethanols erwartet wurde.

In gleicher Weise wie vorangehend beschrieben, aber unter Verwendung unterschiedlicher Aethanol-Konzentrationen im Bereich von 0 - 130 mmol/l, wurden weitere Präparate hergestellt und jeweils die Konzentration an Nitrat-Reduktionsprodukten, gemessen als Nitrit, in der Lösung bestimmt. Die gemessenen Nitrit-Konzentrationen sind in Fig. 2 in Abhängigkeit der verwendeten Aethanol-Konzentration graphisch dargestellt. Zum Vergleich sind ebenfalls die theoretisch möglichen Nitrit-Konzentrationen eingezeichnet, welche unter der Annahme berechnet wurden, dass bei der Umsetzung mit Aethanol aus Nitrat ausschliesslich in der Lösung als Nitrit messbare Nitrat-Reduktionsprodukte gebildet werden.

Fig. 2 zeigt, dass die gemessenen Nitrit-Konzentrationen mit steigender Aethanol-Konzentration nicht linear zunehmen. Bei Aethanol-Konzentrationen unterhalb 45 mmol/l wurden nur geringe Mengen an Nitrat-Reduktionsprodukten gefunden; die gemessenen Nitrit-Konzentrationen lagen deutlich unter dem Wert von 0,1 mg Nitrit pro ml, der den Beginn der therapeutischen Wirksamkeit reflektiert. Ab einer Aethanol-Konzentration von 45 mmol/l erfolgte jedoch ein steiler Anstieg der Nitrit-Konzentration mit zunehmender Aethanol-Konzentration, was eine Uebersättigung der Lösung mit nitrosen Gasen anzeigt.

Beispiel 3 (Haar-Test)

In analoger Weise zu Beispiel 1 wurden unter Verwendung der in Tabelle 2 angegebenen Salpetersäure- und Aethanol-Konzentrationen mehrere Präparate hergestellt und deren Wirkung auf menschliches Haar untersucht. Zu diesem Zweck wurde blondes menschliches Haar mit einer Lösung von 1 Vol.-% Aceton in Wasser und dann mit Wasser gewaschen und getrocknet. Das so entfettete Haar wurde fein zerschnitten. Anschliessend wurden jeweils 10 mg des Haares zu 1 ml des Präparates zugegeben und die Wirkung anhand der einsetzenden Gelbfärbung und einer eventuellen Zersetzung visuell beurteilt. Die Farbänderung signalisiert eine chemische Reaktion mit dem Präparat, durch die das Gewebe die Lebensfähigkeit verliert, aber seine anatomische Struktur bis zu einer allfälligen Zersetzung weitgehend beibehält. Die Wirkung des Handelspräparates Solcoderm wurde zum Vergleich ebenfalls geprüft.

Tabelle 2

| (menschliches Haar) | | | |
|---|---|---|---|
| Salpetersäure-Konzentration (mol/l) | Aethanol-Konzentration (mmol/l) | Beginn der Gelbfärbung nach | Zersetzung |
| 6,4 | 0 | 2 h | nach 18 h |
| 5,4 | 0 | 8 h | nach 18 h |
| 6,4 | 26 | 2 min. | nach 18 h |
| 5,4 | 26 | 2 min. | nach 18 h |
| 6,4 | 60 | 2 min. | nein |
| 5,4 | 60 | 2 min. | nein |
| 4,9 | 60 | 2 min. | nein |
| 5,4 | 86 | 2 min. | nein |
| Solcoderm | | 2 min. | nein |

Proben ohne Aethanol-Zusatz ergaben erst mit grosser Verzögerung eine Gelbfärbung und führten anschliessend zur Zersetzung der Haare; die Gelbfärbung wurde durch die Verringerung der Salpetersäure-Konzentration zusätzlich verzögert. Die Verwendung von 26 mmol Aethanol pro l in 6,4 M bzw. 5,4 M Salpetersäure bewirkte eine rasche Gelbfärbung, führte aber schliesslich ebenfalls zur Zersetzung der Haare. Die unter Verwendung einer Aethanol-Konzentration von 60 bzw. 86 mmol/l hergestellten Proben ergaben für alle untersuchten Salpetersäure-Konzentrationen eine rasche Gelbfärbung ohne Zersetzung der Haare. Unterschiede in der Schnelligkeit der Gelbfärbung mit diesen Proben waren nicht zu beobachten; der Haar-Test erwies sich für diesen Aspekt als zu wenig sensitiv.

Die durch Umsetzung des Aethanols mit Salpetersäure gebildeten Nitrat-Reduktionsprodukte bewirken somit eine erhebliche Beschleunigung der Reaktion mit dem Gewebe und folglich eine deutlich verbesserte Wirkung. Oberhalb eines gewissen Schwellwertes an Nitrat-Reduktionsprodukten wird zudem die Zersetzung des Gewebes unterdrückt, d.h. die Anwesenheit von Nitrat-Reduktionsprodukten in ausreichender Menge gewährleistet die Fixierung der anatomischen Struktur (Mumifizierung) und vermindert die Gefahr von Nebenwirkungen.

Beispiel 4 (Pepsin-Test)

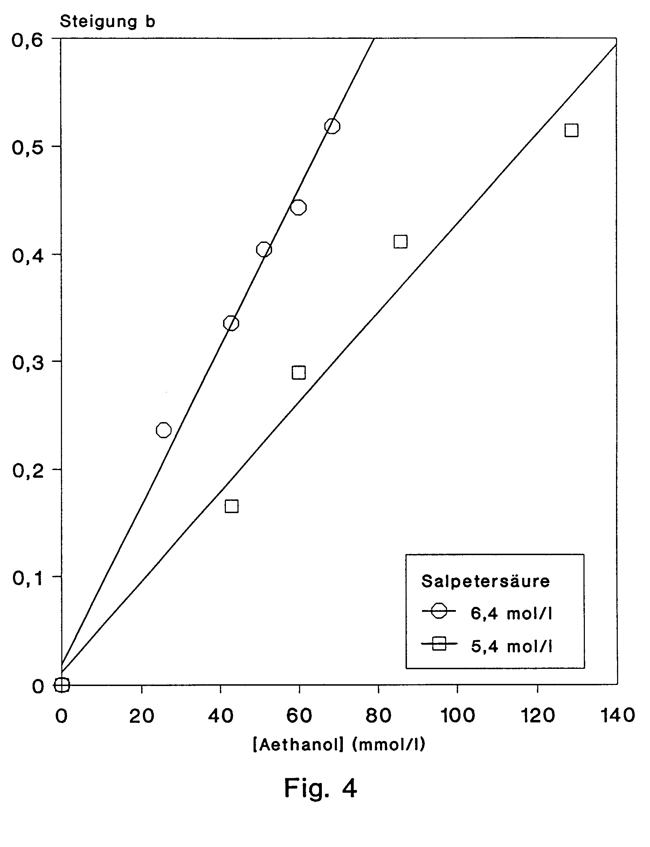

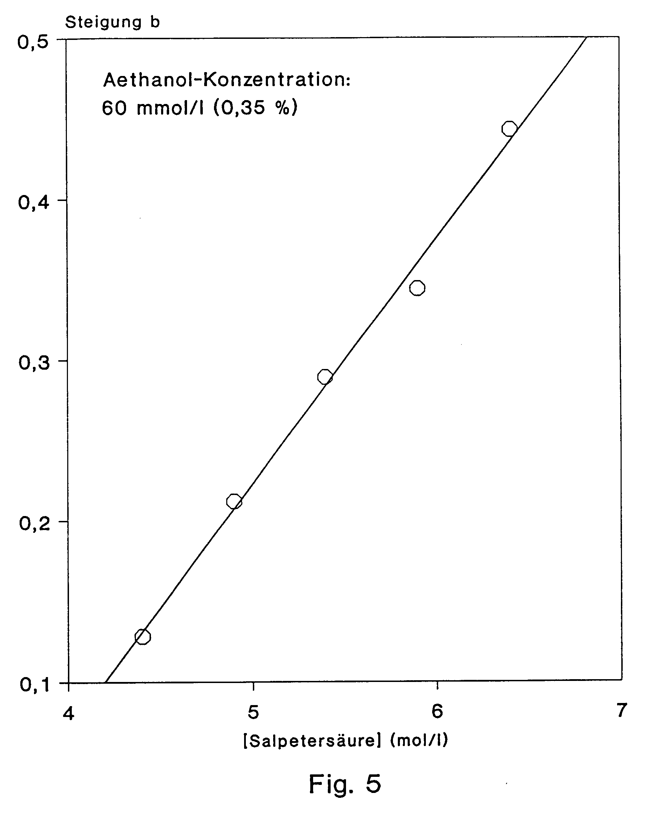

In analoger Weise zu Beispiel 1 wurden unter Verwendung der in Tabelle 3 sowie in Fig. 4 und 5 angegebenen Salpetersäure- und Aethanol-Konzentrationen mehrere Präparate hergestellt und diese anschliessend mit einer Pepsin-Lösung auf ihre Wirkung geprüft. Der Pepsin-Test wurde als Modell für die erste Reaktion der Mumifizierung (Reaktion mit primären und sekundären Aminogruppen, die sich in einer Verfärbung der Lösung äussert) entwickelt und erlaubt eine genauere Quantifizierung dieser Wirkung als die Gelbfärbung im Haar-Test.

Zur Durchführung des Pepsin-Tests wurden jeweils 100 μl der Probe genau 1 Minute nach dem Oeffnen der Ampulle zu einer Lösung von 1 mg Pepsin in 1 ml Wasser zugegeben. Die einsetzende Reaktion wurde anhand der zeitabhängigen Verfärbung des Gemisches spektrophotometrisch verfolgt. Gemessen wurde jeweils die optische Dichte bei einer Wellenlänge von 438 nm über einen Zeitraum von 300 Sekunden ab Zugabe der Probe zur Pepsinlösung. Die erste Messung erfolgte jeweils 20 Sekunden nach der Zugabe. Die gemessenen optischen Dichten wurden für jede Probe als Kurve mit der Gleichung

$$y = a + bx + cx^2 \tag{1}$$

ausgewertet, wobei y die optische Dichte und x die Zeit in Sekunden bedeuten und die Parameter a, b und c berechnet wurden. Die Schnelligkeit der Verfärbung wird im wesentlichen durch Parameter b - im Rahmen der vorliegenden Erfindung als "Steigung b" bezeichnet - bestimmt, der deshalb als ein Mass für das Wirkungsmodell herangezogen wurde.

Proben Nr. 1 bis 10 ergaben die in Tabelle 3 angegebenen Werte für die Steigung b. Zum Vergleich wurde ebenfalls das Handelspräparat Solcoderm im Pepsin-Test untersucht.

Tabelle 3

| (Pepsin-Test) | | | |
|---|---|---|---|
| Probe Nr. | Salpetersäure-Konzentration (mol/l) | Aethanol-Konzentration (mmol/l) | Steigung b |
| 1 | 6,4 | 0 | 0 |
| 2 | 4,9 | 60 | 0,212 |
| 3 | 5,9 | 60 | 0,344 |
| 4 | 6,4 | 51 | 0,404 |
| 5 | 5,4 | 86 | 0,411 |
| 6 | 6,4 | 60 | 0,443 |
| 7 | 3,4 | 129 | 0,223 |
| 8 | 4,4 | 129 | 0,340 |
| 9 | 5,4 | 129 | 0,514 |
| 10 | 6,4 | 129 | 0,705 |
| Solcoderm | | | 0,316 |

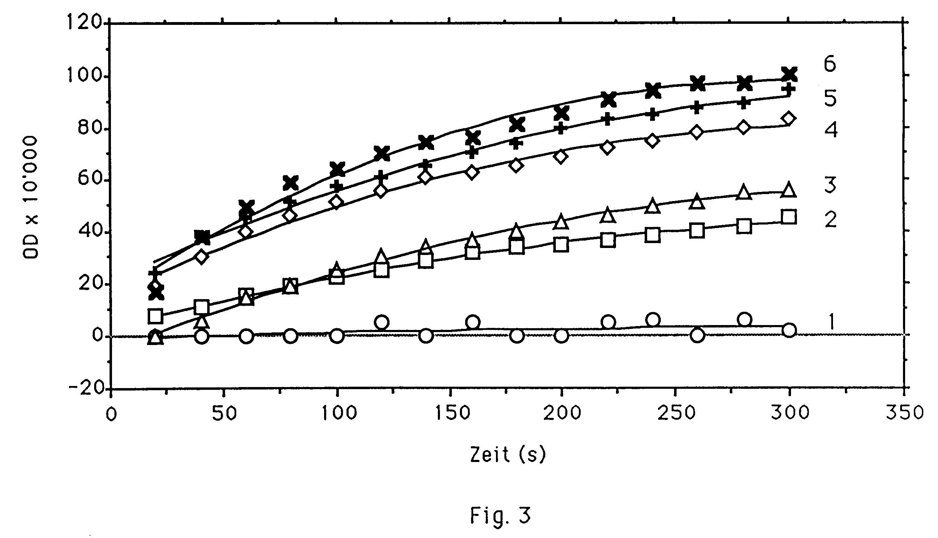

In Fig. 3 sind die für Proben Nr. 1 bis 6 gemessenen optischen Dichten (OD) in Abhängigkeit der Zeit graphisch dargestellt.

Das Ergebnis der Probe Nr. 1 bestätigt, dass ohne Aethanol-Zusatz hergestellte Präparate unwirksam sind. Proben Nr. 2 bis 6 zeigten eine gute Wirkung, welche offenbar sowohl mit steigender Salpetersäure-Konzentration als auch mit steigender Aethanol-Konzentration zunimmt.

Fig. 4 zeigt in graphischer Darstellung die Werte der Steigung b von Präparaten, die unter Verwendung variabler Aethanol-Konzentrationen und einer konstanten Salpetersäure-Konzentration von 5,4 bzw. 6,4 mol/l hergestellt wurden. Wie aus Fig. 4 ersichtlich ist, waren die Werte der Steigung b annähernd proportional zu den verwendeten Aethanol-Konzentrationen. Präparate mit 5,4 M Salpetersäure ergaben einen weniger steilen Kurvenverlauf; dieser Effekt kann jedoch durch eine entsprechende Erhöhung der Aethanol-Konzentration kompensiert werden.

Fig. 5 zeigt in graphischer Darstellung die Werte der Steigung b von Präparaten, die mit einer konstanten Aethanol-Konzentration von 60 mmol/l und einer variablen Salpetersäure-Konzentration im Bereich von 4,4 - 6,4 M hergestellt wurden. Wie der Figur entnommen werden kann, nehmen die Werte der Steigung b in diesem Konzentrationsbereich annähernd linear zu mit steigender Salpetersäure-Konzentration.

Beispiel 5 (Test auf menschlicher Haut)

Zum Zwecke der Beurteilung der Nebenwirkungen auf normale menschliche Haut wurden zwei Präparate, die gemäss Beispiel 1 aus Salpetersäure (6,4 bzw. 5,4 mol/l) und Aethanol (60 bzw. 86 mmol/l) hergestellt wurden, sowie zum Vergleich entsprechende und stärker verdünnte Salpetersäurelösungen ohne Aethanol-Zusatz und das Handelspräparat Solcoderm auf der Unterseite des Vorderarmes aufgetragen und 5 Minuten einwirken gelassen. Proben mit Salpetersäure-Konzentrationen von 5,4 mol/l und weniger wurden in einer Menge von 40 µl aufgetragen, die übrigen in einer Menge von 20 µl. Die Wirkung wurde über einen Zeitraum von 36 Stunden beobachtet und ergab das in Tabelle 4 aufgeführte Bild.

Tabelle 4

| (menschliche Haut, Unterseite des Vorderarmes) | | | | |
|---|---|---|---|---|
| | sofort nach Einwirkung | nach 1 h | nach 7 h | nach 36 h |
| 6,4 M Salpetersäure (20 µl) | starke Rötung | | | offene Wunde |
| | Bildung einer grossen Blase | | Blase offen | |

Tabelle 4 (fortgesetzt)

| (menschliche Haut, Unterseite des Vorderarmes) | | | |
|---|---|---|---|
| sofort nach Einwirkung | nach 1 h | nach 7 h | nach 36 h |
| 5,4 M Salpetersäure (40 µl) | starke Rötung | | |
| | Bildung einer Blase | Blase bleibt geschlossen | |
| 4,4 M Salpetersäure (40 µl) | mässige Rötung | | |
| | Bildung einer kleinen Blase | Blase bleibt geschlossen | Blase verschwunden |
| 3,4 M Salpetersäure (40 µl) | mässige Rötung | | |
| | Bildung einer kleinen Blase | Blase bleibt geschlossen | Blase verschwunden |
| 2,4 M Salpetersäure (40 µl) | leichte Rötung | sehr leichte Rötung | keine Rötung |
| 1,4 M Salpetersäure (40 µl) | sehr leichte Rötung | keine Rötung | |
| Solcoderm (20 µl) | starke Rötung mit bräunlichen Rändern | | |
| | Bildung einer Blase | Blase bleibt geschlossen | |
| 6,4 M Salpetersäure + 60 mmol/l Aethanol (20 µl) | deutliche Braunfärbung mit leichter Rötung | | |
| | Bildung einer kleinen Blase | Blase bleibt geschlossen | Blase verschwunden |
| 5,4 M Salpetersäure + 86 mmol/l Aethanol (40 µl) | deutliche Braunfärbung mit leichter Rötung | | |
| | sehr geringe Blasenbildung | Blase verschwunden | |

5,4 M Salpetersäure ergab trotz Auftragung des doppelten Volumens geringere Nebenwirkungen als 6,4 M Salpetersäure und führte insbesondere nicht zur Bildung einer offenen Wunde. Mit weiter abnehmender Salpetersäure-Konzentration wurde eine weitere Reduktion der Nebenwirkungen beobachtet. Insbesondere trat bei 2,4 M und 1,4 M Salpetersäure keine Blasenbildung mehr auf. Solcoderm ergab eine ähnliche Reaktion wie 5,4 M Salpetersäure. Die beiden unter Aethanol-Zusatz hergestellten Präparate führten im Vergleich zu den entsprechenden, reinen Salpetersäurelösungen zu deutlich geringeren Nebenwirkungen, die zudem rasch abklangen, d.h. die durch Umsetzung mit Aethanol in diesen Präparaten gebildeten Nitrat-Reduktionsprodukte unterdrückten die Nebenwirkungen der Salpetersäure auf normales Gewebe weitgehend. Beide Präparate erwiesen sich auch als deutlich besser als Solcoderm.

Das aus 5,4 M Salpetersäure und Aethanol in einer Konzentration von 86 mmol/l hergestellte Präparat zeigte ähnlich geringe Nebenwirkungen wie reine 2,4 M Salpetersäure. Es ermöglicht eine sichere Anwendung, da die Nebenwirkungen selbst dann vergleichsweise gering bleiben, wenn der Gehalt an Nitrat-Reduktionsprodukten infolge unsachgemässer Handhabung ganz oder teilweise abgebaut wird. Ein Kontrollversuch mit einer Probe (20 µl), die erst 24 Stunden nach Oeffnen der Ampulle auf die Haut aufgetragen wurde und deren Wirkung ebenfalls über einen Zeitraum von 36 Stunden beobachtet wurde, ergab eine starke Rötung mit bräunlichen Flecken sowie die Bildung von Blasen, die geschlossen blieben. Da das Risiko von Nebenwirkungen bei Verringerung der Salpetersäure-Konzentration noch weiter abnimmt, eignen sich solche Präparate auch zur Behandlung von grossflächigen Haut- und Schleimhauterkrankungen.

Beispiel 6

Ein in analoger Weise zu Beispiel 1 und unter Verwendung einer Salpetersäure-Konzentration von 5,4 mol/l und einer Aethanol-Konzentration von 86 mmol/l hergestelltes Präparat (gemessener Nitritgehalt: 3,20 mg/ml) wurde über einen Zeitraum von 30 Minuten ab dem Oeffnen der Ampulle auf seine Wirkung im Pepsin-Test untersucht. Der Pepsin-Test wurde durchgeführt wie in Beispiel 4 beschrieben. Die Ergebnisse sind in Tabelle 5 zusammengestellt.

Tabelle 5

| Zeit nach Oeffnen (min.) | Steigung b | Steigung b in % des Anfangswertes |
|---|---|---|
| 0,3 | 0,506 | 100 |
| 1 | 0,483 | 95 |
| 3 | 0,392 | 77 |
| 6 | 0,289 | 57 |
| 10 | 0,239 | 47 |
| 23 | 0,221 | 44 |
| 30 | 0,167 | 33 |
| 54 | 0,102 | 20 |

Die Auftragung des Präparates innerhalb weniger Minuten bis zu einer Stunde, vorzugsweise innerhalb einer halben Stunde, nach Oeffnen der Ampulle garantiert die therapeutische Wirksamkeit und die weitgehende Nebenwirkungsfreiheit.

Beispiel 7

Unter Verwendung der in Tabelle 6 angegebenen Aethanol- und Salpetersäurekonzentrationen wurden, wie in Beispiel 1 beschrieben, mehrere Präparate hergestellt und diese nach dem Verschmelzen der Ampullen bei 22°C aufbewahrt. Nach unterschiedlicher Lagerzeit wurde mittels gaschromatographischer Analyse der Essigsäuregehalt der Präparate bestimmt. Kontrollmessungen mittels einer enzymatischen Methode (Acetat-Testkit von Boehringer) ergaben im Rahmen der Messgenauigkeit übereinstimmende Ergebnisse.

Tabelle 6

| Aethanol-Konzentration (mmol/l) | Salpetersäure-Konzentration (mol/l) | Essigsäure-Konzentration (mg/ml) | Lagerzeit bei 22°C |
|---|---|---|---|
| 43 | 5,4 | 0,55 | 15 Monate |
| 43 | 6,4 | 0,93 | 19 Monate |
| 60 | 4,9 | 0,61 | 21 Tage |
| 60 | 4,9 | 0,59 | 16 Monate |
| 60 | 5,4 | 0,93 | 21 Tage |
| 60 | 5,4 | 0,90 | 16 Monate |
| 60 | 6,4 | 1,63 | 27 Tage |
| 60 | 6,4 | 1,56 | 16 Monate |
| 86 | 5,4 | 1,57 | 23 Stunden |
| 86 | 5,4 | 1,55 | 27 Tage |
| 86 | 5,4 | 1,63 | 13 Monate |
| 129 | 5,4 | 2,92 | 14 Monate |
| 129 | 6,4 | 4,63 | 21 Monate |

Die Ergebnisse bestätigen, dass die Essigsäure-Konzentration auch bei langer Lagerung konstant bleibt und dass eine Erhöhung der eingesetzten Aethanol-Konzentration zu einer höheren Essigsäure-Konzentration führt. Die gemessenen Essigsäure-Konzentrationen sind jedoch niedriger als die eingesetzten Aethanol-Konzentrationen. Mittels eines Bariumchlorid-Tests konnte Kohlendioxid als weiteres Umsetzungsprodukt von Aethanol nachgewiesen werden. In den ausreagierten Lösungen konnten weder Aethanol noch andere Umsetzungsprodukte nachgewiesen werden, das heisst Aethanol wurde vollständig zu Essigsäure und Kohlendioxid umgesetzt.

Beispiel 8

In analoger Weise zu Beispiel 1 wurde 5,4 M Salpetersäure mit Aethanol in einer Menge von 86 mmol pro l Lösung versetzt und die Lösung in mehrere Ampullen abgefüllt. Die ampullierten Lösungen wurden 14 Tage bzw. 1 Monat bei unterschiedlicher Temperatur gelagert und dann auf ihren Gehalt an Essigsäure und Nitrat-Reduktionsprodukten untersucht. Die Ergebnisse sind in Tabelle 7 aufgeführt. Sie bestätigen, dass die Lagertemperatur im untersuchten Bereich keinen signifikanten Einfluss auf die Zusammensetzung des Präparates hat.

Tabelle 7

| Lagerbedingungen | Nitrit-Konzentration (mg/ml) | Essigsäure-Konzentration (mg/ml) |
|---|---|---|
| 14 Tage bei 22°C | 2,35 | 1,42 |
| 30°C | 2,55 | 1,42 |
| 40°C | 2,32 | 1,39 |
| 50°C | 2,47 | 1,42 |
| 1 Monat bei 22°C | 2,37 | 1,43 |
| 30°C | 2,22 | 1,44 |
| 40°C | 2,27 | 1,42 |
| 50°C | 2,81 | 1,40 |

Beispiel 9 (Behandlung von Gemein- und Plantarwarzen)

Zur Durchführung einer klinischen Studie zur Behandlung von Gemein- oder Plantarwarzen wurden nach der in Beispiel 1 beschriebenen Methode die folgenden Präparate hergestellt:

Tabelle 8

| Präparat | Aethanol-Konzentration (mmol/l) | Salpetersäure-Konzentration (mol/l) |
|---|---|---|
| A | 60 | 5,4 |
| B | 86 | 5,4 |
| C | 129 | 4,4 |

Insgesamt wurden 26 Warzen (22 Patienten) mit einem der Präparate A - C behandelt, und bei Patienten mit zwei ähnlichen Warzenarten wurde ferner eine Warze zum Vergleich mit Solcoderm behandelt. Die Applikation der Präparate erfolgte mit einem dünnen, porösen Kunststoffstäbchen, indem das Präparat mit dem Kunststoffstäbchen tröpfchenweise auf der Läsion verteilt und durch leichtes Sticheln in die Läsion eingearbeitet wurde. Diese Behandlung wurde jeweils fortgesetzt, bis sich die Läsion gleichmässig gelb verfärbt hatte und/ oder ein leichtes Brennen verspürt wurde. Die Präparate A - C führten jeweils innert etwa 3 bis 5 Minuten zur Verfärbung der Läsion, Solcoderm innert etwa 10 Minuten. Ebenso trat ein Wärmegefühl und Prickeln bei den Präparaten A - C schneller auf (innert etwa 5 bis 10 Minuten) als bei Solcoderm (innert 15 bis 30 Minuten). Die Behandlungsdauer mit den Präparaten A - C war somit deutlich kürzer, und zudem war die Schmerzempfindung bezüglich Intensität und Dauer geringer als bei der Behandlung mit Solcoderm. Im Abstand von 4 bis 8 Tagen wurde die Behandlung ein- oder mehrmals wiederholt, sofern die Läsion nicht vollständig geheilt war. Die Ergebnisse sind in Tabelle 9 zusammengestellt.

Tabelle 9

| Indikation/Präparat | Total Fälle | Heilung | | |
|---|---|---|---|---|
| | | nach 1 Behandlung | nach 2 Behandlung | unvollständig nach 2 Behandlungen |
| Gemeine Warzen | | | | |
| B | 4 | - | 2 | 2 |
| C | 7 | 3 | 2 | 2 |
| Plantarwarzen | | | | |
| A | 5 | 2 | 2 | 1 |
| B | 6 | 1 | 3 | 2 |
| C | 4 | 1 | 1 | 2 |

Von den in Tabelle 9 aufgeführten Fällen, in denen die Läsion nach 2 Behandlungen noch nicht vollständig geheilt war, war eine der mit Präparat B behandelten Gemeinwarzen nach der 3. Behandlung, eine der mit Präparat B behandelten Plantarwarzen nach der 3. und die andere nach der 4. Behandlung und eine der mit Präparat C behandelten Plantarwarzen nach der 3. Behandlung vollständig abgeheilt. Die anderen Fälle waren bei der Erstellung des Zwischenberichts noch in Behandlung.

Mit dem Vergleichspräparat wurden 4 Gemeinwarzen und 2 Plantarwarzen behandelt, wovon 2 Gemeinwarzen und 1 Plantarwarze nach einer Behandlung und 2 Gemeinwarzen und 1 Plantarwarze nach der zweiten Behandlung abgeheilt waren.

Beispiel 10 (Fusspilz-Behandlung)

In einer Fallstudie wurde ein Patient mit Fusspilzbefall an beiden Füssen mit einem erfindungsgemässen Präparat behandelt, welches nach der in Beispiel 1 beschriebenen Methode aus 5,4 M Salpetersäure und 86 mmol Aethanol pro I Lösung hergestellt wurde.

Ein Fuss wies starken Pilzbefall zwischen der vierten und fünften Zehe mit Hautablösungen und starker Rötung auf. Beim Spreizen der Zehen riss die dünne Resthaut auf, was zu Blutungen führte. Nach erfolgloser Behandlung mit einem Oxiconazol-Präparat (Oceral, Roche), wurden ca. 100 µl des erfindungsgemässen Präparates auf die Läsion aufgetragen, wobei der Patient während wenigen Sekunden ein kurzes, stechendes Brennen an den verletzten Hautstellen verspürte und sich gewisse eng begrenzte Bereiche um die verletzte Hautstelle braun verfärbten. Der grosse Rest der befallenen Haut und der umliegenden gesunden Haut zeigte keine Verfärbung oder nur geringe Gelbfärbung. 8 Stunden nach der Behandlung wies die behandelte Haut nach wie vor eine starke Rötung auf, und der Patient verspürte noch einen leichten Druckschmerz, aber keinen Ruheschmerz mehr. Die Läsion wurde in der Folge jeweils über Nacht mit Wundheilungssalbe (Solcoseryl Salbe, Solco Basel AG) behandelt. Nach 3 Tagen waren die für Fusspilz typischen Hautläsionen verschwunden; die Hautoberfläche war hart und straff. Nach 7 Tagen war die befallene Stelle vollständig ausgeheilt, und es trat keine Rekurrenz auf.

Der andere Fuss wies leichten Pilzbefall ohne Hautrisse auf. Die befallenen Stellen wurden mit dem erfindungsgemässen Präparat behandelt, wobei keine Schmerzen auftraten. Nach 7 Tagen war die Läsion vollkommen abgeheilt, und es trat ebenfalls keine Rekurrenz auf.

Beispiel 11 (Nagelpilzbehandlung)

In einer weiteren Fallstudie wurde ein Patient mit Nagelpilz (Totalbefall der Ring-, Mittel- und Zeigefingernägel der rechten Hand) mit einem erfindungsgemässen Präparat behandelt, welches nach der in Beispiel 1 beschriebenen Methode aus 3,4 M Salpetersäure und 129 mmol Aethanol pro I Lösung hergestellt wurde. Vor dieser Behandlung waren die befallenen Nägel mit einem Amorolfin-Präparat (Loceryl-Lack, Roche) behandelt worden, wobei weder eine Abnahme des Pilzbefalls noch ein Nagelwachstum beobachtet werden konnte.

Das erfindungsgemässe Präparat wurde auf die befallenen Nägel aufgetragen, wobei innert einer Minute eine deutliche Gelbbraun-Färbung auftrat. Der Patient verspürte keine Schmerzen, ausser einem leichten Brennen während etwa 3 Minuten an einer Stelle, an der die Haut am Uebergang vom Nagelbett zur normalen Haut eine kleine Verletzung aufwies. Nach 3 Tagen bröckelten Nagelteile, die besonders stark infiziert waren, ab. Die Behandlung mit dem erfindungsgemässen Präparat wurde ca. alle 7 Tage wiederholt. Nach 14 Tagen war bereits eine deutliche Verbesserung der Nägel (geringere Zerstörung) zu beobachten. Nach 2 Monaten war sichtbar, dass die Nägel wieder nachwachsen; der hintere, an das Nagelbett angrenzende Nagelteil war jedoch infolge Pilzbefalls im Nagelbett selber nach wie vor befallen. Das erfindungsgemässe Präparat eignet sich somit zur Behandlung oberflächlicher Nagelpilz-Infektionen; bei Infektion des Nagelbettes muss die Therapie jedoch zusammen mit einem oralen Antimykotikum erfolgen.

**Patentansprüche**

1. Präparat auf der Basis einer wässrigen, Nitrat-Reduktionsprodukte enthaltenden Salpetersäurelösung zur lokalen Behandlung von oberflächlichen Haut-und Schleimhautveränderungen und von oberflächlichen Infektionen der Haut und Schleimhaut, dadurch gekennzeichnet, dass das Präparat aus 1 - 5,5 M wässriger Salpetersäure, Nitrat-Reduktionsprodukten in einer Konzentration, die 0,1 bis 6 mg Nitrit pro ml Lösung entspricht, und $C_1$-$C_5$-Alkansäure in einer Konzentration von höchstens 170 mmol pro l Lösung besteht.

2. Präparat nach Anspruch 1 zur Behandlung von hyperplastischen Haut- und Schleimhautveränderungen, dadurch gekennzeichnet, dass es eine Salpetersäure-Konzentration von 4 - 5,5 M aufweist.

3. Präparat nach Anspruch 1 zur Behandlung von Pilzerkrankungen der Haut und Schleimhaut, dadurch gekennzeichnet, dass es eine Salpetersäure-Konzentration von 1 - 4 M aufweist.

4. Präparat nach einem der Ansprüche 1 bis 3, dadurch gekennzeichnet, dass die Konzentration an Nitrat-Reduktionsprodukten 1 bis 5 mg Nitrit pro ml Lösung entspricht.

5. Präparat nach Anspruch 4, dadurch gekennzeichnet, dass die Konzentration an Nitrat-Reduktionsprodukten 2 bis 4 mg Nitrit pro ml Lösung entspricht.

6. Präparat nach einem der Ansprüche 1 bis 5, dadurch gekennzeichnet, dass die Konzentration an $C_1$-$C_5$-Alkansäure mindestens 5 mmol pro l beträgt.

7. Präparat nach Anspruch 6, dadurch gekennzeichnet, dass die Konzentration an $C_1$-$C_5$-Alkansäure 9 bis 90 mmol pro l beträgt.

8. Präparat nach einem der Ansprüche 1 bis 7, dadurch gekennzeichnet, dass es als $C_1$-$C_5$-Alkansäure Essigsäure enthält.

9. Präparat nach einem der Ansprüche 1 bis 8, dadurch gekennzeichnet, dass es in gasdicht verschlossenem Gefäss, vorzugsweise in verschlossener Ampulle vorliegt.

10. Präparat nach Anspruch 9, dadurch gekennzeichnet, dass das Verhältnis des Flüssigkeitsvolumens zum Gesamtvolumen des Gefässes höchstens 1:2, vorzugsweise 1:10 bis 1:5, beträgt.

11. Verfahren zur Herstellung eines Präparates gemäss den Ansprüchen 1 bis 10, dadurch gekennzeichnet, dass man 1 - 5,5 M wässrige Salpetersäure mit einem primären $C_1$-$C_5$-Alkanol in einer Menge von 45 bis 170 mmol pro l Salpetersäure reagieren lässt.

12. Verfahren nach Anspruch 11, dadurch gekennzeichnet, dass man 60 bis 90 mmol des primären $C_1$-$C_5$-Alkanols pro l Salpetersäure verwendet.

13. Verfahren nach Anspruch 11 oder 12, dadurch gekennzeichnet, dass man als primäres $C_1$-$C_5$-Alkanol Aethanol verwendet.

14. Verfahren nach einem der Ansprüche 11 bis 13, dadurch gekennzeichnet, dass man die Salpetersäure und das primäre $C_1$-$C_5$-Alkanol bei einer Temperatur unterhalb der Umsetzungstemperatur mischt und das Gemisch in gasdicht verschlossenem Gefäss, vorzugsweise in verschlossenen Ampullen, auf Umsetzungstemperatur oder höhere Temperatur erwärmt.

15. Verfahren nach Anspruch 14, dadurch gekennzeichnet, dass man die Salpetersäure bei einer Temperatur von höchstens 10°C mit Aethanol mischt und das Gemisch in gasdicht verschlossenem Gefäss auf 20 bis 40°C, vorzugsweise auf Raumtemperatur, erwärmt.

**Claims**

1. Preparation based on an aqueous solution of nitric acid, containing nitrate reduction products, for the local treatment of superficial changes in skin and mucous membrane and of superficial infections of the skin and mucous membrane, characterized in that the preparation comprises 1 - 5.5 M aqueous nitric acid, nitrate reduction products in a concentration corresponding to from 0.1 to 6 mg of nitrite per ml of solution, and $C_1$-$C_5$-alkanolic acid in a concentration of not more than 170 mmol per l of solution.

2. Preparation according to Claim 1 for the treatment of hyperplastic changes in skin and mucous membrane, characterized in that it has a nitric acid concentration of 4 - 5.5 M.

3. Preparation according to Claim 1 for the treatment of fungal diseases of the skin and mucous membrane, characterized in that it has a nitric acid concentration of 1 - 4 M.

4. Preparation according to one of Claims 1 to 3, characterized in that the concentration of nitrate reduction products corresponds to from 1 to 5 mg of nitrite per ml of solution.

5. Preparation according to Claim 4, characterized in that the concentration of nitrate reduction products corresponds to from 2 to 4 mg of nitrite per ml of solution.

6. Preparation according to one of Claims 1 to 5, characterized in that the concentration of $C_1$-$C_5$-alkanoic acid is at least 5 mmol per l.

7. Preparation according to Claim 6, characterized in that the concentration of $C_1$-$C_5$-alkanoic acid is from 9 to 90 mmol per l.

8. Preparation according to one of Claims 1 to 7, characterized in that it contains acetic acid as $C_1$-$C_5$-alkanoic acid.

9. Preparation according to one of Claims 1 to 8, characterized in that it is present in a container with a gastight closure, preferably in a sealed ampoule.

10. Preparation according to Claim 9, characterized in that the ratio of the liquid volume to the total volume of the container is not more than 1:2, preferably from 1:10 to 1:5.

11. Process for the production of a preparation according to Claims 1 to 10, characterized in that 1 - 5.5 M aqueous nitric acid is reacted with a primary $C_1$-$C_5$-alkanol in a quantity of from 45 to 170 mmol per l of nitric acid.

12. Process according to Claim 11, characterized in that from 60 to 90 mmol of the primary $C_1$-$C_5$-alkanol are used per l of nitric acid.

13. Process according to Claim 11 or 12, characterized in that ethanol is used as primary $C_1$-$C_5$-alkanol.

14. Process according to one of Claims 11 to 13, characterized in that the nitric acid and the primary $C_1$-$C_5$-alkanol are mixed at a temperature below the reaction temperature and the mixture in a container with a gastight closure, preferably in sealed ampoules, is heated to reaction temperature or above.

15. Process according to Claim 14, characterized in that the nitric acid is mixed with ethanol at a temperature of not more than 10°C and the mixture in a container with a gastight closure is heated to from 20 to 40°C, preferably to room temperature.

**Revendications**

1. Préparation à base d'une solution aqueuse d'acide nitrique contenant des produits de réduction de nitrate pour le traitement local d'altérations superficielles de la peau et des muqueuses et d'infections superficielles de la peau et des muqueuses, caractérisée en ce qu'elle consiste en acide nitrique aqueux 1 à 5,5 M, produits de réduction de nitrate à une concentration qui correspond à 0,1 à 6 mg de nitrite par ml de solution et un acide alcanoïque en $C_1$

à $C_5$ à une concentration d'au plus 170 mmoles par litre de solution.

2. Préparation selon la revendication 1 pour le traitement d'altérations hyperplastiques de la peau et des muqueuses, caractérisée en ce qu'elle présente une concentration en acide nitrique de 4 à 5,5 M.

3. Préparation selon la revendication 1 pour le traitement de mycoses de la peau et des muqueuses, caractérisée en ce qu'elle présente une concentration en acide nitrique de 1 à 4 M.

4. Préparation selon l'une quelconque des revendications 1 à 3, caractérisée en ce que la concentration en produits de réduction de nitrate correspond à 1 à 5 mg de nitrite par ml de solution.

5. Préparation selon la revendication 4, caractérisée en ce que la concentration en produits de réduction de nitrate correspond à 2 à 4 mg de nitrite par ml de solution.

6. Préparation selon l'une quelconque des revendications 1 à 5, caractérisée en ce que la concentration en acide alcanoïque en $C_1$ à $C_5$ est d'au moins 5 mmoles par litre.

7. Préparation selon la revendication 6, caractérisée en ce que la concentration en acide alcanoïque en $C_1$ à $C_5$ est de 9 à 90 mmoles par litre.

8. Préparation selon l'une quelconque des revendications 1 à 7, caractérisée en ce qu'elle contient, comme acide alcanoïque en $C_1$ à $C_5$, l'acide acétique.

9. Préparation selon l'une quelconque des revendications 1 à 8, caractérisée en ce qu'elle se trouve dans un récipient fermé, étanche aux gaz, de préférence dans une ampoule fermée.

10. Préparation selon la revendication 9, caractérisée en ce que le rapport du volume de liquide au volume total du récipient est au plus de 1:2, de préférence de 1:10 à 1:5.

11. Procédé de fabrication d'une préparation selon les revendications 1 à 10, caractérisé en ce que l'on fait réagir de l'acide nitrique aqueux 1 à 5,5 M avec un alcanol primaire en $C_1$ à $C_5$ en une quantité de 45 à 170 mmoles par litre d'acide nitrique.

12. Procédé selon la revendication 11, caractérisé en ce qu'on utilise de 60 à 90 mmoles de l'alcanol primaire en $C_1$ à $C_5$ par litre d'acide nitrique.

13. Procédé selon la revendication 11 ou 12, caractérisé en ce qu'on utilise comme alcanol primaire en $C_1$ à $C_5$ l'éthanol.

14. Procédé selon l'une quelconque des revendications 11 à 13, caractérisé en ce qu'on mélange l'acide nitrique et l'alcanol primaire en $C_1$ à $C_5$ à une température inférieure à la température de réaction et que l'on chauffe le mélange à la température de réaction ou à une température supérieure dans un récipient fermé, étanche aux gaz, de préférence dans des ampoules fermées.

15. Procédé selon la revendication 14, caractérisé en ce qu'on mélange l'acide nitrique avec l'éthanol à une température de 10°C au plus et que l'on chauffe le mélange à une température de 20 à 40°C, de préférence à la température ordinaire, dans un récipient fermé, étanche aux gaz.

Fig. 1

Fig. 2

Fig. 3

EP 0 630 650 B1

Fig. 4

Fig. 5